# EUROPÄISCHE PATENTANMELDUNG

(11) **EP 3 889 967 A1**
(43) Veröffentlichungstag der Anmeldung: **06.10.2021**
(21) Anmeldenummer: 20167127.8
(22) Anmeldetag: 31.03.2020
(51) Int. Cl.: G16H 30/20, G16H 40/67

(54) **TELERADIOLOGISCHES SYSTEM UND VERFAHREN**

(71) Anmelder: XCoorp GmbH, 1160 Wien (AT)
(72) Erfinder: WINKLER, Benjamin, 8244 Schäffern (AT); PECK, Michael, 1160 Wien (AT)
(74) Vertreter: Schmitt-Nilson Schraud Waibel Wohlfrom Patentanwälte Partnerschaft mbB

(57) **Zusammenfassung**

Ein erfindungsgemäßes teleradiologisches System umfasst wenigstens ein Diagnostic Gate (DG) mit einer Schnittstelle zu einer bilderzeugenden Modalität (M) einer lokalen Untersuchungseinrichtung (LU) oder zu einem Bildarchivierungs- und Kommunikationssystem (PACS) zur Speicherung von Patienten-Bilddaten, einer lokalen Untersuchungseinrichtung (LU) oder zu einem Radiologieinformationssystem (RIS/KIS) zur Speicherung von medizinischen und administrativen Daten und zur Prozesssteuerung; eine Datenspeicherungs- und Verteileinrichtung (DC) mit einer Servereinheit (S); und einer Verteileinheit (MDR); und wenigstens einen Empfänger (ER, IR, Al). Das Diagnostic Gate (DG) ist so ausgebildet ist, dass es unbearbeitete oder unbefundete Studient, von einer bilderzeugenden Modalität (M) oder von einem Bildarchivierungs- und Kommunikationssystem (PACS) empfängt, und an die Datenspeicherungs- und Verteileinrichtung (DC) versendet. Die Servereinheit (S) ist zur Abspeicherung und Zur-Verfügungsstellung der von dem Diagnostic Gate (DG) empfangenen unbearbeiteten oder unbefundeten Studien, und zur Abspeicherung und Zur-Verfügungsstellung der von der Verteileinheit (MDR) empfangenen, bearbeiteten oder befundeten Studien ausgebildet.

## Beschreibung

Die vorliegende Erfindung betrifft ein teleradiologisches System und Verfahren, insbesondere ein teleradiologisches Fernbefundungs-, Fernbearbeitungs- und Datenmanagement-System und -Verfahren.

In den letzten Jahren sind teleradiologische Verfahren und Systeme entstanden, die es erlauben, eine Befundung nicht lokal im Krankenhaus oder im Institut durchführen zu müssen, sondern diese entfernt von der Modalität an einem Remote-Arbeitsplatz durchführen zu können.

Ein teleradiologisches Verfahren ist z. B. aus der DE 10 2014 001 601 A1 bekannt. Bei dieser ist das Befundungszentrum entfernt von dem Krankenhaus vorgesehen. Der Datenaustausch erfolgt zwischen Krankenhaus und dem Server des Befundungszentrums.

In der Praxis hat es sich gezeigt, dass die Anbindung von zusätzlichen Krankenhäusern mit radiologischen Abteilungen oder Radiologie-Praxen an solche Systeme aufwändig und teuer ist.

Es ist daher die Aufgabe der vorliegenden Erfindung, ein kostengünstiges teleradiologisches System und Verfahren anzugeben, mit denen eine beliebige Anzahl von lokalen Radiologie-Einheiten kostengünstig fernbefundet und fernbearbeitet werden können und dass auch die Fernbefundungs- und Fernbearbeitungsmöglichkeiten erweitert werden.

Diese Aufgaben werden durch den Gegenstand der unabhängigen Patentansprüche gelöst. Vorteilhafte Weiterbildungen ergeben sich aus den abhängigen Patentansprüchen.

Ein erfindungsgemäßes radiologisches System umfasst die Merkmale des unabhängigen Patentanspruchs 1.

Ein erfindungsgemäßes radiologisches Verfahren umfasst die Merkmale des unabhängigen Patentanspruchs 14.

Mit dem erfindungsgemäßen teleradiologischen System und Verfahren können eine beliebige Anzahl von Diagnostic Gates von lokalen Untersuchungseinheiten, also radiologischen Abteilungen von Krankenhäusern oder radiologischen Praxen, an ein erfindungsgemäßes radiologisches System, insbesondere an die Datenspeicherungs- und Verteileinrichtung angeschlossen werden, ohne dass teure zusätzliche Schnittstellen geschaffen werden müssen.

Es ist lediglich ein Diagnostic Gate pro lokaler radiologischer Untersuchungseinheit vorzusehen. Dieses Diagnostic Gate übernimmt alle Funktionen des Datenaustauschs, insbesondere des Versands der unbearbeiteten oder unbefundeten Studien an die Datenspeicherungs- und Verteileinrichtung und die entsprechende Rücksendung des entsprechenden Befunds an die lokale Untersuchungseinrichtung, insbesondere zur Anzeige auf der Bedienkonsole oder zur Abspeicherung auf dem PACS oder RIS/KIS.

Unter dem RIS ist dabei das Radiologieinformationssystem einer Radiologiepraxis und unter dem KIS das Krankenhausinformationssystem, das auch als HIS = Hospital Information System bezeichnet werden kann, zu verstehen.

Wenn derzeit eine neue lokale Untersuchungseinrichtung an einen bestehenden Teleradiologie-Server abgeschlossen werden soll, so werden Kosten in der Größenordnung von 40.000 EUR fällig.

Die Kosten für ein erfindungsgemäßes Diagnostic Gate liegen weit darunter, es können somit durch die Erfindung Kosten in signifikantem Umfang eingespart werden.

Des Weiteren können eine Vielzahl von Empfängern Bestandteil des erfindungsgemäßen teleradiologischen Systems, insbesondere der Datenspeicherungs- und Verteileinrichtung sein, diese können z.B. als interne radiologische Bearbeitungs- oder Befundungs-Arbeitsplätze ausgebildet sein.

Des Weiteren können als automatische Vorbearbeitungs- oder Vorbefundungseinheit AI ausgebildete Empfänger Bestandteil des erfindungsgemäßen teleradiologischen Systems, insbesondere der Datenspeicherungs- und Verteileinrichtung sein, die eine Vorbearbeitung oder Vorbefundung durchführen. Die automatische Vorbearbeitungs- oder Vorbefundungseinheit kann dabei insbesondere selbstlernend ausgebildet, also insbesondere mit künstlicher Intelligenz KI bzw. artificial intelligence AI ausgestattet sein.

Ebenso können eine Vielzahl von als externe radiologische Fernbearbeitungs- oder Fernbefundungs-Arbeitsplätze an das erfindungsgemäße teleradiologische System, insbesondere an dessen Datenspeicherungs - und Verteileinrichtung angeschlossen werden bzw. mit diesem kommunizieren. Das Anbinden von neuen externen teleradiologischen Befundungs- oder Bearbeitungsplätzen ist auf einfache Weise und ohne große Kosten möglich.

Die unbearbeiteten oder unbefundeten Studien können somit an eine Vielzahl von solchen Empfängern gesendet, und von diesen bearbeitet oder befundet werden, und diese können die Bearbeitungsergebnisse oder Befunde an die Verteileinheit zurücksenden.

Somit ist die Anzahl der lokalen radiologischen Untersuchungseinrichtungen als auch die Anzahl der Empfänger, die eine Bearbeitung oder Befundung durchführen können, beliebig skalierbar.

Durch die Servereinheit S der Datenspeicherungs- und Verteileinrichtung ist sichergestellt, dass alle Studien, insbesondere unbearbeitete oder unbefundete Studien, bearbeitete oder befundete Studien, von einer automatischen Vorbearbeitungs- oder Vorbefundungseinheit vorbearbeitete oder vorbefundete Studien und anschließend von einem lokalen Radiologen in der lokalen Untersuchungseinrichtung endbearbeitete oder endbefundete Studien in der Servereinheit S zentral, verliersicher und wiederauffindbar abgespeichert werden.

Den zuweisenden Ärzten oder Bearbeitern oder Befundern oder auch Patienten bzw. deren Arbeitsplätzen kann ein Zugang zu solchen zentral abgespeicherten Studien ermöglicht werden.

Durch das erfindungsgemäße teleradiologische System und Verfahren werden somit der notwendige Funktionsumfang als auch andere wichtige Teile der Datensicherheit und Übertragungstechnik bereitgestellt, wie in den nachfolgenden Ausführungsformen noch beschrieben werden wird.

Das erfindungsgemäße teleradiologische System und Verfahren kann auf einfache Weise bei bestehenden lokalen Untersuchungseinrichtungen nachgerüstet werden, indem untersuchungseinrichtungsseitig einfach ein Diagnostic Gate vorgesehen und mit der dortigen Modalität, dem dortigen PACS und/oder dem dortigen RIS/KIS verbunden wird. Dieses Diagnostic Gate sorgt dann für den nötigen Datenaustausch mit der Datenspeicherungs- und Verteileinrichtung, die insbesondere entfernt von der lokalen Untersuchungseinrichtung vorgesehen ist.

Diese Nachrüstung eines Diagnostic Gates erfordert ansonsten keinerlei Änderungen der bestehenden Modalität, der bestehenden lokalen Bedienkonsole, des bestehenden PACS und des bestehenden RIS/KIS. Es muss nur eine Datenverbindung zwischen der Modalität, dem PACS und/oder dem RIS/KIS zu dem Diagnostic Gate hergestellt werden.

Das Diagnostic Gate kann auf einem ohnehin schon auf der lokalen Untersuchungseinrichtung vorhandenen Computer als Software installiert werden, die mit der Modalität, dem PACS oder RIS/KIS verbunden ist bzw. wird und in Datenverbindung mit der Speicherungs- und Verteileinrichtung steht bzw. diese aufbaut.

Insbesondere kann das Diagnostic Gate als sogenannte virtuelle Maschine VM eines solchen, bereits vorhandenen Computers ausgebildet sein.

Es ist selbstverständlich ebenfalls möglich, das Diagnostic Gate als zusätzliche Computereinheit auf Seite der lokalen Untersuchungseinrichtung vorzusehen, die wiederum mit der lokalen Bedienkonsole, dem PACS und dem RIS/KIS einerseits und der Datenspeicherungs- und Verteileinrichtung, insbesondere deren Servereinheit andererseits verbunden ist.

Die Work Flows der internen und externen radiologischen Bearbeitungs- und Befundungs-Arbeitsplätze können dabei beibehalten werden.

Zudem kann durch das Vorsehen eines als automatische Vorbearbeitungs- oder Vorbefundungseinheit ausgebildeten Empfängers die Möglichkeit geschaffen werden, eine automatische und bedienerlose Vorbearbeitung und Vorbefundung durchzuführen, und Vorbefundungsergebnisse und Vorbefunde zu erhalten, welche die Endbefundung erleichtern, die üblicherweise durch den Radiologen in der lokalen Untersuchungseinrichtung durchgeführt wird.

Gemäß einem weiteren Aspekt können die Diagnostic Gates von verschiedenen lokalen Untersuchungseinrichtungen von einer zentralen Infrastruktur konfiguriert, gewartet und supported werden. Alle Updates können, wie bei einem Command-and-Control-Server (CC-Server) zentral zur Verfügung gestellt werden, sodass sich die Diagnostic Gates selbstständig updaten.

Dadurch, dass das Diagnostic Gate die Daten bzw. Studien im Standarddatenformat DICOM und insbesondere auch im Standarddatenformat HL-7 erhält und auch die Bearbeitungsergebnisse und Befunde in diesem Datenformat zurückerhält und in der lokalen Bedieneinrichtung zur Verfügung stellt, ist das erfindungsgemäße teleradiologische System und Verfahren einfach integrierbar.

Der Kommunikationsaufbau von jedem Diagnostic Gate zu der Datenspeicherungs- und Verteileinrichtung, insbesondere zu deren Servereinheit, kann selbstständig erfolgen und ist einfach herzustellen. Eine Möglichkeit einer solchen Datenverbindung ist diejenige des VPN, die meist ohnehin auf Seiten der lokalen Untersuchungseinrichtung vorhanden ist. Diese Datenverbindung kann als eine extrem starke, sich selbstständig neu aufbauende und sich selbst überwachende IPSec IKE v2 Internetverbindung ausgebildet sein, die mit einem eigenen Router-Cluster zur Einwahl VRRP und einer eigenen CA Certification Authority realisiert werden kann, um höchsten Ansprüchen zu genügen.

Erfindungsgemäß ist das Erstellen von Bearbeitungsergebnissen und Befunden in ähnlich schneller Zeit möglich, als wenn eine lokale Bearbeitung oder Befundung durch einen Radiologen an der Bedienkonsole der Modalität erfolgen würde.

Die Datenspeicherungs- und Verteileinrichtung kann als Datencenter-Rechenzentrum oder als Data Center ausgebildet sein.

Das HL7 Datenformat kann optional zu einer Studie dazugehören, muss also nicht unbedingt vorgesehen sein. Zusätzlich zum Studienmaterial können optional Informationen im HL7 Datenformat übermittelt werden.

Wie in den vorteilhaften Ausbildungen noch erläutert werden wird, kann das Diagnostic Gate selbst eine Anonymisierung oder Pseudonymisierung der versendeten Daten, insbesondere der versendeten unbearbeiteten oder unbefundeten Studien vornehmen.

Zudem kann sowohl zwischen der lokalen Untersuchungseinrichtung bzw. den lokalen Untersuchungseinrichtungen und der Datenspeicherungs- und Verteileinrichtung, insbesondere deren Servereinheit als auch zwischen der Datenspeicherungs- und Verteileinrichtung und den externen radiologischen Befundungs- oder Bearbeitungsplätzen, den Arbeitsplätzen der zuweisenden Ärzte, Bearbeiter oder Befunder oder den Arbeitsplätzen der Patienten, die ebenfalls an die Datenspeicherungs- und Verteileinrichtung angeschlossen werden können, eine verschlüsselte Datenkommunikation erfolgen, um höchsten Sicherheits- und Datenschutzstandards zu genügen.

Das Bearbeitungsergebnis oder der Befund kann dabei in einem Standard-Datenformat vorliegen, insbesondere als pdf, xml, dicom embedded pdf, hl7 oder auch Klartext txt.

Die Datenspeicherungs- und Verteileinrichtung, insbesondere deren Servereinheit und deren Verteileinheit können die Historie, z. B. die Anzahl und die jeweiligen Zeitpunkte der versendeten Studien tracken und speichern, was eine Basis für Auswertungen darstellt.

Das erfindungsgemäße teleradiologische System und Verfahren bietet auch die Möglichkeit einer Zweitmeinung. In diesem Falle ist schon ein Bearbeitungsergebnis oder ein Befund durch einen Radiologen erstellt und im PACS oder RIS/KIS abgespeichert worden, jedoch ist das Bearbeitungsergebnis/der Befund nicht eindeutig. In diesem Falle ist die zugrunde liegende Studie als unbearbeitete oder unbefundete Studie anzusehen, die von dem Diagnostic Gate erfindungsgemäß zur Bearbeitung oder Befundung an die Datenspeicherungs- und Verteileinrichtung gesendet wird, wie hier beschrieben.

Der kleinste Einheit der vorliegenden Erfindung ist wie folgt: Das oder die Diagnostic Gates der jeweils zugeordneten lokalen Untersuchungseinrichtung, die Datenspeicherungs- und Verteileinrichtung mit Servereinheit und mit Verteileinheit und wenigstens ein Empfänger, also z. B. ein interner radiologischer Bearbeitungs- oder Befundungs-Arbeitsplatz und/oder eine interne automatische Vorbefundungs- oder Bearbeitungseinheit. In diesem kleinsten Verwirklichungsrahmen der Erfindung sind weder die übrigen Elemente der lokalen Untersuchungseinrichtung(en) noch evtl. vorgesehene externe radiologische Bearbeitungs- oder Befundungs-Arbeitsplätze Bestandteil des Systems.

Gemäß einer ersten Ausführungsform der Erfindung umfasst das teleradiologische System weiterhin wenigstens eine lokale Untersuchungseinrichtung mit einer bilderzeugenden Modalität; einem Bildarchivierungs- und Kommunikationssystem PACS zur Speicherung von Patienten-Bilddaten, insbesondere im DICOM-Datenformat; einem Radiologieinformationssystem RIS/KIS zur Speicherung von medizinischen und administrativen Daten und zur Prozesssteuerung, insbesondere im HL7-Datenformat; und einer lokalen Bedienkonsole zur Steuerung der Modalität. Das Diagnostic Gate ist ebenfalls Bestandteil der lokalen Untersuchungseinrichtung und so ausgebildet, dass es unbearbeitete oder unbefundete Studien, insbesondere im DICOM-Datenformat, von der bilderzeugenden Modalität oder von dem Bildarchivierungs- und Kommunikationssystem PACS empfängt, ggf. anonymisiert oder pseudonymisiert, und an die Datenspeicherungs- und Verteileinrichtung, insbesondere an die Servereinheit versendet.

Gemäß einer weiteren Ausführungsform ist die Verteileinheit so ausgebildet, dass sie eine Bearbeitung oder einen Befund, nach Erstellung desselben, mit dem Datensatz der unbearbeiteten oder unbefundeten Studie verknüpft, um daraus eine bearbeitete oder befundete Studie zu erstellen.

Gemäß einer weiteren Ausführungsform sind zwei oder mehrere lokale Untersuchungseinrichtungen vorgesehen. Die Verteileinheit umfasst eine Liste von Empfängern, eine Liste von lokalen Untersuchungseinrichtungen oder deren Diagnostic Gates und eine Berechtigungsinformation, welche Empfänger Studien von welchem Diagnostic Gate erhalten dürfen. Die Verteileinheit führt dabei eine Berechtigungsverwaltung durch.

Dadurch ist sichergestellt, dass die entsprechenden Studien nur von denjenigen Empfängern bearbeitet oder befundet werden, die hierfür auch vorgesehen sind.

Gemäß einer weiteren Ausführungsform verfügt die Datenspeicherungs- und Verteileinrichtung über wenigstens einen als internen radiologischen Bearbeitungs- oder Befundungsarbeitsplatz (IR) ausgebildeten Empfänger und/oder über einen als automatische Vorbearbeitungs- oder Vorbefundungseinheit ausgebildeten Empfänger, insbesondere mit künstlicher Intelligenz AI.

Es kann dabei wenigstens ein als externer radiologischer Bearbeitungs- oder Befundungsarbeitsplatz ausgebildeter Empfänger entfernt von der Datenspeicherungs- und Verteileinrichtung vorgesehen sein.

Gemäß einer weiteren Ausführungsform führt in dem Fall, dass die Datenspeicherungs- und Verteileinrichtung über einen als automatische Vorbearbeitungs- oder Vorbefundungseinheit ausgebildeten Empfänger, insbesondere mit künstlicher Intelligenz verfügt, dieser bedienerlos eine Vorbearbeitung oder Vorbefundung durch.

Wenn die automatische Vorbearbeitungs- oder Vorbefundungseinheit eine Vorbefundung durchführt, vergleicht diese wenigstens eine Sequenz von Patienten-Bilddaten einer unbearbeiteten oder unbefundeten Studie automatisiert mit gespeicherten Referenzbildern, und erstellt aus dem Vergleichsergebnis einen Vorbefund.

Wenn die automatische Vorbearbeitungs- oder Vorbefundungseinheit eine Vorbearbeitung durchführt, vermisst diese wenigstens eine Sequenz von Patienten-Bilddaten einer unbearbeiteten oder unbefundeten Studie automatisiert oder führt automatisiert eine Auswertung oder einen Vergleich mit gespeicherten Referenzbildern durch, und stellt ein Vorbearbeitungsergebnis zur Verfügung.

Gemäß einer weiteren Ausführungsform ist die Verteileinheit so ausgebildet, dass sie aus dem von der automatischen Vorbearbeitungs- oder Vorbefundungseinheit erstellten Vorbefund/Vorbearbeitungsergebnis, aus der eindeutigen Datensatz-ID und der unbefundeten oder befundeten Studie eine vorbearbeitete oder vorbefundete Studie erstellt. Die vorbearbeitete oder vorbefundete Studie ist ein Datensatz, mit der eindeutigen Datensatz-ID, mit oder ohne Patientendaten, mit der wenigstens einen Sequenz von Patienten-Bilddaten der unbearbeiteten oder unbefundeten Studie und mit der/dem von der automatischen Vorbearbeitungs- oder Vorbefundungseinheit (AI) erstellten Vorbefund/Vorbearbeitungsergebnis.

Gemäß einer weiteren Ausführungsform ist die Verteileinheit so ausgebildet, dass sie die vorbearbeitete oder vorbefundete Studie an die Servereinheit zur Abspeicherung derselben darin sendet, und/oder dass die vorbearbeitete oder vorbefundete Studie über das Diagnostic Gate an die lokale Untersuchungseinrichtung, insbesondere an die lokale Bedienkonsole zur Endbearbeitung und/oder Endbefundung und/oder zur Abspeicherung in dem PACS und/oder in dem RIS/KIS gesendet wird.

Bei dieser Ausführungsform erfolgt somit das Erstellen der vorbearbeiteten oder vorbefundeten Studie auf der Verteileinheit selbst.

Gemäß einer weiteren Ausführungsform ist die Verteileinheit so ausgebildet ist, dass sie den/das von der automatischen Vorbearbeitungs- oder Vorbefundungseinheit erstellte(n) Vorbefund/Vorbearbeitungsergebnis zusammen mit der eindeutigen Datensatz-ID an die Servereinheit zur Abspeicherung derselben darin sendet, und/oder dass der Vorbefund/ das Vorbearbeitungsergebnis zusammen mit der eindeutigen Datensatz-ID über das Diagnostic Gate an die lokale Untersuchungseinrichtung gesendet wird. In der lokalen Untersuchungseinrichtung wird eine vorbearbeitete oder vorbefundete Studie aus dem Vorbefund/Vorbearbeitungsergebnis, aus der eindeutigen Datensatz-ID und der unbefundeten oder befundeten Studie erstellt und/oder in dem PACS und/oder in dem RIS/KIS abgespeichert. Die vorbearbeitete oder vorbefundete Studie ist ein Datensatz, mit der eindeutigen Datensatz-ID, mit oder ohne Patientendaten, mit der wenigstens einen Sequenz von Patienten-Bilddaten der unbearbeiteten oder unbefundeten Studie und mit der/dem von der automatischen Vorbearbeitungs- oder Vorbefundungseinheit erstellten Vorbefund/Vorbearbeitungsergebnis. Wenigstens der Vorbefund/das Vorbearbeitungsergebnis wird auf der lokalen Bedienkonsole angezeigt. Anhand dessen kann eine Endbefundung durchgeführt werden.

Bei dieser Ausführungsform erfolgt somit das Erstellen der vorbearbeiteten oder vorbefundeten Studie durch die lokalen Untersuchungseinrichtung(en).

Gemäß einer weiteren Ausführungsform ist die lokale Untersuchungseinrichtung, insbesondere die lokale Bedienkonsole so ausgebildet, dass sie eine Endbearbeitung oder einen Endbefund, nach Erstellung desselben, mit dem Datensatz der vorbearbeiteten oder vorbefundeten Studie verknüpft, um daraus eine endbearbeitete oder endfundete bzw. befundete Studie zu erstellen, und dass sie die endbearbeitete oder endbefundete Studie über das Diagnostic Gate an die Servereinheit der Datenspeicherungs- und Verteileinrichtung zur Abspeicherung derselben darin versendet, und/oder dass sie die endbearbeitete oder endbefundete Studie in dem PACS und/oder in dem RIS/KIS abspeichert.

Das Vorliegen des Vorbearbeitungsergebnisses oder der vorbefundeten Studie erleichtert die Endbearbeitung und die Endbefundung und die Erstellung des Endbearbeitungsergebnisses / des Endbefunds.

Gemäß einer weiteren Ausführungsform ist die Servereinheit, insbesondere Serverfarm, zur Abspeicherung und Zur-Verfügungsstellung der unbearbeiteten oder unbefundeten Studien, und/oder der bearbeiteten oder befundeten Studien, und/oder der vorbearbeiteten oder vorbefundeten Studien und/oder der endbearbeiteten oder endbefundeten Studien, insbesondere im DICOM- und/oder HL7-Datenformat für Befunde/Zuweisungen, ausgebildet.

Die Servereinheit, insbesondere Serverfarm, kann dabei insbesondere so ausgebildet sein, dass sie Zugriff auf unbearbeitete oder unbefundete Studien, und/oder auf bearbeitete oder befundete Studien, und/oder auf vorbearbeitete oder vorbefundete Studien und/oder auf endbearbeitete oder endbefundete Studien, insbesondere im DICOM- und/oder HL7-Datenformat für Befunde/Zuweisungen, von einem Arbeitsplatz eines zuweisenden Arztes oder anderen Befunders oder Behandlers oder Bearbeiters oder einem Patientenarbeitsplatz aus erlaubt.

Bei dem Patientenarbeitsplatz handelt es sich üblicherweise um den Heimcomputer des untersuchten Patienten.

Gemäß einer weiteren Ausführungsform ist das Diagnostic Gate oder die Verteileinheit so ausgebildet ist, dass es unbearbeitete oder unbefundete Studien anonymisiert oder pseudonymisiert. Dabei kann das Diagnostic Gate oder die Verteileinheit insbesondere so ausgebildet sein, dass beim Pseudonymisieren einer unbearbeiteten oder unbefundeten Studie die Patientendaten und die wenigstens eine Sequenz von Patienten-Bilddaten verschlüsselt werden, unter unverschlüsselter Beibehaltung der eindeutigen Datensatz-ID.

Durch das Anonymisieren oder Pseudonymisieren, das auf dem Diagnostic Gate oder auf der Verteileinheit erfolgen kann, können eine hohe Datensicherheit und der erforderliche Datenschutz gewährleistet werden.

Gemäß einer weiteren Ausführungsform wird die unbearbeitete oder unbefundete oder die endbearbeitete oder endbefundete Studie vor dem Versenden durch das Diagnostic Gate verschlüsselt, und anschließend durch die Servereinheit entschlüsselt wird.

Gemäß einer weiteren Ausführungsform wird die bearbeitete oder befundete Studie oder die vorbearbeitete oder vorbefundete Studie vor dem Versenden an das Diagnostic Gate durch die Servereinheit verschlüsselt, und anschließend durch das Diagnostic Gate entschlüsselt wird.

Gemäß einer weiteren Ausführungsform wird die unbearbeitete oder unbefundete Studie vor dem Versenden der unbearbeiteten oder unbefundeten Studie an den wenigstens einen als externen radiologischen Befundungsarbeitsplatz ausgebildeten Empfänger verschlüsselt, und durch diesen entschlüsselt.

Gemäß einer weiteren Ausführungsform wird die bearbeitete oder befundete Studie vor dem Zurücksenden durch den wenigstens einen als externen radiologischen Befundungsarbeitsplatz ausgebildeten Empfänger verschlüsselt, und anschließend entschlüsselt.

Durch eine solche Verschlüsselung kann die Datensicherheit erhöht und den Erfordernissen des Datenschutzes entsprochen werden. Die Verschlüsselung kann dabei als 256-Bit-SSL-Verschlüsselung erfolgen.

Gemäß einer weiteren Ausführungsform ist/sind die bilderzeugende Modalität und/oder das PACS so ausgebildet oder programmiert, dass die unbearbeiteten oder unbefundeten Studien automatisch an das Diagnostic Gate versendet werden. Durch eine solche Push-Einspeisung der unbearbeiteten oder unbefundeten Studien wird ein automatisierter und besonders effektiver Workflow ermöglicht.

Die unbearbeiteten oder unbefundeten Studien können alternativ von einem Benutzer, insbesondere der lokalen Bedienkonsole, manuell an das Diagnostic Gate versendet werden. Somit kann alternativ zu der Push-Einspeisung das Einspeisen der unbearbeiteten oder unbefundeten Studien an das Diagnostic Gate auch benutzergesteuert erfolgen.

Gemäß einer weiteren Ausführungsform weist die Datenspeicherungs- und Verteileinrichtung weiterhin eine Diagnostic Gate-Informationseinheit DGIS aufweist, zur Abspeicherung von Metadaten, Ergebnissen und weiteren Informationen. Die Diagnostic Gate-Informationseinheit kann dabei insbesondere mit der Servereinheit und der Verteileinheit verbunden sein.

Diese Diagnostic Gate-Informationseinheit erlaubt den Zugang zu wichtigen Kenndaten für Auswertungszwecke, wie Metadatenergebnissen, Warnhinweisen, Bewertungen, Feedback usw.

Gemäß einer weiteren Ausführungsform ist die bilderzeugende Modalität als CT (Computertomografie)-, als MRT (Magnetresonanztomographie)-, als Ultraschall-, als EKG-, als Endoskopie-, als PetCT-, als NUK (Nuklearmedizinisches), als Röntgen-, als Durchleuchtungs oder als Mammografiescreening-Vorrichtung ausgebildet ist.

Bei dem teleradiologischen System handelt es sich um ein teleradiologisches Fernbefundungs-, Fernbearbeitungs- und Datenmanagementsystem.

Die Erfindung betrifft auch ein Verfahren wie in dem unabhängigen Verfahrensanspruch 14 angegeben.

Dieses erfindungsgemäße Verfahren entspricht in verfahrensmäßiger Hinsicht dem teleradiologischen System gemäß dem unabhängigen Anspruch 1.

Die oben mit Bezug auf das teleradiologische System angegebenen Vorteile und Ausführungsformen treffen in gleicher Weise für das erfindungsgemäße Verfahren zu und werden nicht noch einmal wiederholt.

Die Anmelderin behält sich ausdrücklich das Recht vor, abhängige Verfahrensansprüche analog den abhängigen Systemansprüchen 2 bis 13, jeweils in verfahrensmäßiger Entsprechung, im Prüfungsverfahren oder im Rahmen einer Teilanmeldung hinzuzufügen.

Einer Ausführungsform des Verfahrens ist in Anspruch 15 angegeben.

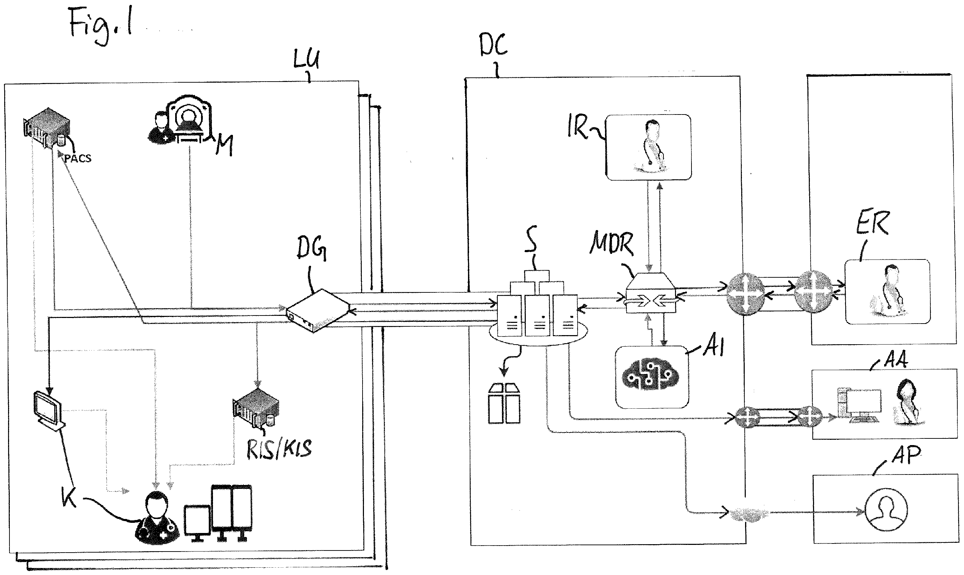

Die Erfindung ist nachfolgend anhand eines Ausführungsbeispiels mit Bezug auf die beiliegende Figur 1 näher erläutert.

Figur 1 zeigt eine schematische Darstellung eines teleradiologischen Fernbefundungs-, Fernvorbefundungs- und/oder Fernbearbeitungs- und Datenmanagementsystems, im Folgenden kurz teleradiologisches System genannt.

Der Datenfluss, insbesondere die Richtung, in welcher die jeweiligen Studien und die jeweiligen Befunde und Bearbeitungsergebnisse zwischen den einzelnen Einheiten des teleradiologischen Systems übermittelt werden, ist in Figur 1 mittels der Pfeilen dargestellt.

Das teleradiologische System umfasst eine lokale Untersuchungseinrichtung LU, die eine bilderzeugende Modalität M, ein Bildarchivierungs- und Kommunikationssystem PACS zur Speicherung von Patienten-Bilddaten, insbesondere im DICOM-Datenformat, nachfolgend kurz PACS genannt; ein Radiologie-Informationssystem bzw. KrankenhausInformationssystem RIS/KIS zur Speicherung von medizinischen und administrativen Daten und zur Prozesssteuerung, insbesondere im HL-7-Datenformat, nachfolgend kurz Radiologie-Informationssystem RIS/KIS oder auch nur RIS/KIS genannt; eine lokale Bedienkonsole K zur Steuerung der Modalität M sowie ein Diagnostic Gate DG.

Das Diagnostic Gate hat eine Schnittstelle zu der bilderzeugenden Modalität M, zu dem PACS und zu dem RIS/KIS und zur lokalen Bedienkonsole K und ist mit diesen Einheiten über entsprechende Datenverbindungen verbunden.

Die bilderzeugende Modalität M kann als CT (Computertomographie)-, als MRT (Magnetresonanztomographie)-, als Ultraschall-, als EKG (Elektrokardiogramm)-, als Endoskopie-, als PetCT-, als NUK (nuklearmedizinisches)-, als Röntgen-, als Durchleuchtungs- oder als Mammographiescreening-Vorrichtung ausgebildet sein.

Die lokale Untersuchungseinrichtung LU wie in Figur 1 gezeigt, kann in radiologischen Abteilungen von Krankenhäusern oder Kliniken oder in speziellen radiologischen Arztpraxen vorhanden sein. In Figur 1 ist durch drei hintereinander angeordnete Rechtecken angedeutet, dass das teleradiologische System eine Vielzahl von solchen lokalen Untersuchungseinrichtungen LU umfassen kann, die allesamt über die Elemente bilderzeugende Modalität M, PACS, RIS/KIS, lokale Bedienkonsole K und Diagnostic Gate DG verfügen.

Das teleradiologische System umfasst des Weiteren eine zentrale Datenspeicherungs- und Verteileinrichtung DC, das in einem Rechenzentrum oder als Datacenter vorliegen kann, die eine Servereinheit S, insbesondere eine Serverfarm, eine Verteileinheit bzw. einen Medical Data Router MDR, einen als internen radiologischen Befundungsarbeitsplatz IR ausgebildeten Empfänger und einen als automatische Vorbefundungs- oder Bearbeitungseinheit AE, insbesondere mit künstlicher Intelligenz ausgebildeten Empfänger umfasst.

Die Servereinheit S ist dabei mittels entsprechenden Datenverbindungen mit dem Diagnostic Gate DG der lokalen Untersuchungseinrichtung, in dem Falle, dass mehrere lokale Untersuchungseinrichtungen LU vorgesehen sind, mit jedem Diagnostic Gate DG der vorhandenen lokalen Untersuchungseinrichtungen LU verbunden. Diese Datenverbindungen können kabelgebunden oder auch drahtlos ausgebildet sein.

Die Datenspeicherungs- und Verteileinrichtung DC ist dabei insbesondere entfernt von der oder den lokalen Untersuchungseinrichtungen LU angeordnet, und insbesondere nicht in dem Krankenhaus / der Klinik oder der Radiologie-Praxis in welcher die lokale Untersuchungseinrichtung vorgesehen ist. Des Weiteren sind entfernt von der oder den lokalen Untersuchungseinrichtungen LU und entfernt von der Datenspeicherungs- und Verteileinrichtung DC als externe Radiologie-Arbeitsplätze ER ausgebildete Empfänger vorgesehen. Zudem kann noch wenigstens ein Arbeitsplatz eines zuweisenden Arztes AA oder eines anderen Befunders oder Behandlers oder Bearbeiters sowie einen Patienten-Arbeitsplatz AP vorgesehen sein.

Die Datenspeicherungs- und Verteileinrichtung DC ist mittels geeigneter Datenverbindungen mit der Servereinheit S und über die Servereinheit S mit dem Diagnostic Gate DG bzw. den Diagnostic Gates DG der lokalen Untersuchungseinrichtung(en) verbunden. Des Weiteren ist die Datenspeicherungs- und Verteileinrichtung DC empfängerseitig mit dem oder den internen radiologischen Befundungs-Arbeitsplätzen IR, mit der oder den automatischen Vorbefundungs- oder Befundungseinheiten AI und mit dem oder den externen radiologischen Befundungs-Arbeitsplätzen ER verbunden.

Die Datenverbindungen zu der Servereinheit S, zu dem oder den internen radiologischen Befundungs-Arbeitsplätzen IR, zu der oder den automatischen Vorbefundungs- oder Bearbeitungseinheiten AI sind dabei insbesondere kabelgebunden ausgeführt. Die Datenverbindung zwischen der Verteileinheit MDR und dem oder den externen radiologischen Befundungs-Arbeitsplätzen ER sind dabei insbesondere wenigstens teilweise drahtlos ausgeführt, also z. B. über ein Medical Gate.

Der oder die Arbeitsplätze von zuweisenden Ärzten, anderen Befundern und Behandlern oder Bearbeitern AA und der oder die Patienten-Arbeitsplätze AP sind mittels entsprechenden Datenverbindungen direkt mit der Servereinheit S verbunden, wobei diese Datenverbindungen wenigstens teilweise drahtlos ausgeführt sein können. Bei dem oder den Arbeitsplätzen von zuweisenden Ärzten, anderen Befundern, Behandlern oder Bearbeitern AA kann die drahtlose Datenverbindung als sogenanntes Medical Gate ausgeführt sein. Bei dem oder den Patienten-Arbeitsplätzen AP kann die Datenverbindung wenigstens teilweise als Internetdatenverbindung (web access) ausgeführt sein.

Im Betrieb empfängt das Diagnostic Gate DG unbearbeitete oder unbefundete Studien, insbesondere im DICOM-Datenformat von der bilderzeugenden Modalität M oder von dem PACS und versendet diese an die Datenspeicherungs- und Verteileinrichtung DC, insbesondere an deren Servereinheit S.

Eine unbefundete Studie ist ein Datensatz mit einer eindeutigen Datensatz-ID, mit oder ohne Patientendaten und mit wenigstens einer Sequenz von Patienten-Bilddaten. Hier können die unbearbeiteten oder unbefundeten Studien entweder automatisch von der bilderzeugenden Modalität M und/oder von dem PACS an das Diagnostic Gate DG versendet werden. Alternativ dazu können die unbearbeiteten oder unbefundeten Studien auch von einem Benutzer der lokalen Bedienkonsole K manuell an das Diagnostic Gate DG versendet werden.

Die Servereinheit S dient zur Abspeicherung und zur Verfügungstellung der von der Diagnostic Gate DG empfangenen unbearbeiteten oder unbefundeten Studien, insbesondere im DICOM und/oder HL-7-Datenformat, und zur Abspeicherung und zur Verfügungstellung der von der Verteileinheit MDR empfangenen, bearbeiteten oder befundeten Studien, ebenfalls insbesondere im DICOM- und/oder HL-7-Datenformat.

Die Verteileinheit MDR überprüft, nach Empfang einer entsprechenden unbearbeiteten oder unbefundeten Studie, welcher Empfänger E die Berechtigung hat, eine unbefundete oder befundete Studie zu erhalten, die von einer bestimmten lokalen Untersuchungseinrichtung LU bzw. von deren Diagnostic Gate DG stammt und sendet diese unbefundete oder befundete Studie an diesen berechtigen Empfänger E, also an den internen radiologischen Bearbeitungs- oder Befundungs-Arbeitsplatz IR, an den externen radiologischen Bearbeitungs- oder Befundungs-Arbeitsplatz IR oder an die automatische Vorbearbeitungs- oder Bevorbefundungseinheit AI.

Zunächst wird nun die Funktion und der Verfahrensablauf beschrieben für den Fall, dass die Verteileinheit MDR die unbefundete oder die befundete Studie an den internen oder externen radiologischen Bearbeitungs- oder Befundungs-Arbeitsplatz IR bzw. ER sendet.

An diesem internen oder externen radiologischen Bearbeitungs- oder Befundungs-Arbeitsplatz IR bzw. ER wird basierend auf der wenigstens einen Sequenz von Patienten-Bilddaten, üblicherweise basierend wenigstens auf einem Teil der Patientendaten, insbesondere Alter und Geschlecht, durch den an diesem Arbeitsplatz arbeitenden Teleradiologen eine Befundung oder eine Bearbeitung durchgeführt, und es wird ein Befund oder ein Bearbeitungsergebnis erstellt.

Anschließend wird der Befund oder das Bearbeitungsergebnis, zusammen mit der eindeutigen Datensatz-ID, aber ohne die wenigstens eine Sequenz von Patienten-Bilddaten und ggf. auch ohne Patientendaten, von dem internen oder externen radiologischen Bearbeitungs- oder Befundungs-Arbeitsplatz IR, ER zurück an die Verteileinheit MDR gesendet.

Wenn nun die Verteileinheit MDR die unbefundete oder die befundete Studie an die automatische Vorbearbeitungs- oder Vorbefundungseinheit AI sendet, so erfolgt dort eine Vorbearbeitung oder Vorbefundung.

Bei einer Vorbefundung wird wenigstens eine Sequenz der Patienten-Bilddaten der unbearbeiteten oder unbefundeten Studie automatisiert mit gespeicherten Referenzbildern verglichen, und aus dem Vergleichsergebnis wird ein Vorbefund erstellt.

Wenn eine Vorbearbeitung durchgeführt wird, wird eine Sequenz von Patienten-Bilddaten der unbearbeiteten oder unbefundeten Studie automatisiert vermessen, automatisiert eine Auswertung oder ein Vergleich mit gespeicherten Referenzbildern durchgeführt, und es wird ein Vorbearbeitungsergebnis erzeugt.

Der durch die automatische Vorbearbeitungs- oder Vorbefundungseinheit AI der erstellte Vorbefund oder das erstellte Vorbearbeitungsergebnis wird anschließend mit der eindeutigen Datensatz-ID, aber ohne die Sequenz von Patienten-Bilddaten und ggf. auch ohne die Patientendaten, an die Verteileinheit MDR zurückgesendet.

In einer ersten Ausführungsform erstellt nun die Verteileinheit MDR aus dem Befund oder dem Bearbeitungsergebnis bzw. aus dem Vorbefund oder dem Vorbearbeitungsergebnis, was ja ebenfalls ein Befund oder ein Bearbeitungsergebnis ist, aus der eindeutigen Datensatz-ID und aus der unbefundeten oder befundeten Studie eine bearbeitete oder befundete Studie (oder eine vorbearbeitete oder vorbefundete Studie, was ja ebenfalls eine bearbeitete oder eine befundete Studie darstellt).

Die bearbeitete oder befundete Studie bzw. die vorbearbeitete oder die vorbefundete Studie ist somit ein Datensatz, der die eindeutige Datensatz-ID, die Patientendaten, insbesondere wenigstens das Geschlecht und das Alter des Patienten, die wenigstens eine Sequenz von Patienten-Bilddaten der unbearbeiteten oder unbefundeten Studie und das erstellte Bearbeitungsergebnis oder den erstellten Befund enthält.

In dem Fall, dass der Empfänger eine automatische Vorbearbeitungs- oder Vorbefundungseinheit AI war, stellt deren Vorbearbeitungsergebnis oder deren Vorbefund das Bearbeitungsergebnis oder den Befund dar.

Die bearbeitete oder die befundete Studie wird nun von der Verteileinheit MDR an die Servereinheit S versendet und dort, insbesondere geordnet nach der Datensatz-ID, abgespeichert.

Anschließend kann die bearbeitete oder befundete Studie über das Diagnostic Gate DG an die lokale Untersuchungseinrichtung LU gesendet werden und dort auf der lokalen Bedienkonsole K angezeigt und in dem PACS und/oder in dem RIS/KIS abgespeichert werden.

In einer anderen Ausführungsform sendet die Verteileinheit MDR den Befund oder das Bearbeitungsergebnis (bzw. den Vorbefund oder das Vorbearbeitungsergebnis) zusammen mit der eindeutigen Datensatz-ID an die Servereinheit S, und dort wird dieser / dieses abgespeichert, insbesondere geordnet nach der eindeutigen Datensatz-ID. Der Befund oder das Bearbeitungsergebnis (bzw. der Vorbefund oder das Vorbearbeitungsergebnis) wird zusammen mit der eindeutigen Datensatz-ID anschließend über das Diagnostic Gate DG an die lokale Untersuchungseinrichtung LU gesendet. Dort, insbesondere in dem PACS, wird nun aus dem Bearbeitungsergebnis oder Befund (bzw. aus dem Vorbearbeitungsergebnis oder dem Vorbefund), aus der eindeutigen Datensatz-ID und aus der unbefundeten oder befundeten Studie eine bearbeitete oder befundete Studie erstellt.

Die bearbeitete oder befundete Studie ist dabei ein Datensatz, mit der eindeutigen Datensatz-ID, mit den Patientendaten, insbesondere dem Geschlecht oder dem Alter des Patienten, und mit der wenigstens einen Sequenz von Patienten-Bilddaten der unbearbeiteten oder unbefundeten Studie und mit der / mit dem vom Empfänger erstellten Bearbeitungsergebnis oder Befund (bzw. Vorbearbeitungsergebnis oder Vorbefund). Das Bearbeitungsergebnis oder der Befund (bzw. das Vorbearbeitungsergebnis oder der Vorbefund) wird auf der lokalen Bedienkonsole K dargestellt und kann in dem PACS und/oder RIS/KIS abgespeichert werden.

Die weitere Funktion bzw. das weitere Verfahren unterscheidet sich, je nach dem ob ein (fertiges) Arbeitsergebnis oder ein (fertiger) Befund vorliegt, der durch einen Radiologen an dem internen oder externen radiologischen Bearbeitungs- oder Befundungs-Arbeitsplatz IR oder ER erstellt worden ist, oder ob (nur) ein Vorbearbeitungsergebnis oder ein Vorbefund vorliegt, der durch eine automatische Vorbearbeitungs- oder Vorbefundungseinheit AI (und nicht durch einen Radiologen) erstellt worden ist.

In dem Fall, dass ein fertiges Bearbeitungsergebnis oder ein fertiger Befund vorliegt, der durch einen Radiologen an dem internen oder externen radiologischen Bearbeitungs- oder Befundungs-Arbeitsplatz IR oder ER erstellt worden ist, und wie oben beschrieben eine bearbeitete oder befundete Studie vorliegt, kann diese dem Radiologen, dem Bearbeiter oder dem Bediener an der lokalen Bedienkonsole K zur Verfügung gestellt werden, zur Besprechung mit dem Patienten.

Ebenso kann diese in der Servereinheit S oder in der lokalen Bedieneinrichtung, insbesondere in dem PACS gespeichert werden für einen späteren Zugriff. Über die Servereinheit S kann diese dem zuweisenden Arzt, einem anderen Befunder oder Behandler oder Bearbeiter AA oder dem Patienten selbst, Patienten-Arbeitsplatz AP, zur Verfügung gestellt werden.

In allen Fällen kann aus dem Bearbeitungsergebnis oder dem Befund eine entsprechende medizinische Maßnahme abgeleitet werden, z. B. dass eine bestimmte Operation erforderlich ist.

Wenn es sich bei der bearbeiteten oder befundeten Studie nun um eine vorbearbeitete oder vorbefundete Studie handelt, die durch eine automatische Vorbearbeitungs- oder Vorbefundungseinheit AI automatisch ohne einen Radiologen erstellt worden ist, erfolgt üblicherweise basierend auf diesem / dieser eine Endbearbeitung oder Endbefundung durch einen Radiologen, der dafür das Vorbearbeitungsergebnis oder den Vorbefund, also gewissermaßen ein Zwischenergebnis, das durch die automatische Vorbearbeitungs- oder Vorbefundungseinheit AI erstellt worden ist, berücksichtigen kann.

Der Vorbefund bzw. das Vorbearbeitungsergebnis wird auf der lokalen Bedienkonsole K angezeigt, und es wird dort üblicherweise eine Endbefundung durch den dort befindlichen Radiologen durchgeführt. Dabei kann insbesondere eine endbefundete Studie erstellt werden.

Dies ist ein Datensatz mit der eindeutigen Datensatz-ID, mit den Patientendaten, insbesondere wenigstens dem Geschlecht und dem Alter des Patienten, der wenigstens einen Sequenz von Patienten-Bildern der unbearbeiteten oder unbefundeten bzw. vorbearbeiteten oder vorbefundeten Studie, insbesondere mit der/dem von der automatischen Vorbearbeitungs- oder Vorbefundungseinheit AI erstellten Vorbefund/Vorbearbeitungsergebnis und mit dem erstellten Endbefund. Diese endbefundete Studie kann in dem PACS und/oder nach Übertragung durch das Diagnostic Gate DG an die Servereinheit S in der Servereinheit S insbesondere wiederfindbar nach der eindeutigen Datensatz-ID abgespeichert werden.

Diese endbefundete Studie kann wiederum bspw. dem zuweisenden Arzt, Befunder oder Behandler oder Bearbeiter an dessen Arbeitsplatz AA oder dem Patienten-Arbeitsplatz AP gesendet werden.

Die Funktion/der Ablauf ist für beide Fälle derselbe (bearbeitete oder befundete Studie durch Radiologen an internem oder externem radiologischen Bearbeitungs- oder Befundungs-Arbeitsplatz IR oder ER erstellt; Vorbearbeitungsergebnis oder Vorbefund automatisch und bedienerlos durch Vorbearbeitungs- oder Vorbefundungseinheit AI erstellt). In dem Fall, dass nur eine vorbearbeitete oder vorbefundete Studie vorliegt, wird diese jedoch üblicherweise noch auf der lokalen Bedieneinrichtung, insbesondere durch einen Radiologen an der lokalen Bedienkonsole K endbefundet, wie oben beschrieben.

Wenn die bearbeiteten oder befundeten Studien zentral auf der Servereinheit S abgespeichert werden, so kann dadurch ein zentraler Zugang für alle zuweisenden Ärzte, Radiologen und Behandler sowie für alle Patienten geschaffen werden, die bei entsprechender Berechtigung, auf die jeweiligen Studien zugreifen können.

In einer Ausführungsform kann das Diagnostic Gate DG oder die Verteileinheit MDR unbearbeitete oder unbefundete Studien anonymisieren oder pseudonymisieren.

Des Weiteren kann der Datenaustausch zwischen dem Diagnostic Gate DG und der Servereinheit S, zwischen der Verteileinheit MDR und den externen radiologischen Bearbeitungs- oder Befundungsarbeitsplatz ER sowie zwischen der Servereinheit S und dem Arbeitsplatz AA des zuweisendes Arztes, Befunders oder Behandlers oder Bearbeiters und dem Patienten-Arbeitsplatz AP verschlüsselt erfolgen.

In dem kleinsten Verwirklichungsrahmen der Erfindung sind nur das Diagnostic Gate DG und die Datenspeicherungs- und Verteileinrichtung DC Bestandteil des teleradiologischen Systems.

Ein Beispiel für eine konkrete unbefundete Studie ist wie folgt: Datensatz ID: M-42-01234, Patientendaten: Männlich, 42 Jahre, Körpergröße 178cm, Gewicht 82 kg; Sequenz von Bilddaten: CT-Aufnahmen des Sprunggelenks rechts in mehreren Schichten.

Erstellter Befund: Sprunggelenk gebrochen, Weber-B-Fraktur
Befundete Studie: Datensatz ID: M-42-01234, Patientendaten: Männlich, 42 Jahre, Körpergröße 178cm, Gewicht 82 kg; Sequenz von Bilddaten: CT-Aufnahmen des Sprunggelenks rechts in mehreren Schichten; Befund: Sprunggelenk gebrochen, Weber-B-Fraktur, als Textdatei

## Patentansprüche

1. Teleradiologisches System, aufweisend:
wenigstens ein Diagnostic Gate (DG) mit einer Schnittstelle zu einer bilderzeugenden Modalität (M) einer lokalen Untersuchungseinrichtung (LU) oder zu einem Bildarchivierungs- und Kommunikationssystem (PACS) zur Speicherung von Patienten-Bilddaten, insbesondere im DICOM-Datenformat, einer lokalen Untersuchungseinrichtung (LU) oder zu einem Radiologieinformationssystem (RIS/KIS) zur Speicherung von medizinischen und administrativen Daten und zur Prozesssteuerung, insbesondere im HL7-Datenformat;
eine Datenspeicherungs- und Verteileinrichtung (DC) mit
einer Servereinheit (S), insbesondere einer Serverfarm; und
einer Verteileinheit (MDR), insbesondere einem Medical Data Router; und
wenigstens einen Empfänger (ER, IR, AI), insbesondere einem radiologischen Befundungsarbeitsplatz (ER, IR) oder einer automatischen Vorbefundungs- oder Bearbeitungseinheit (AI), insbesondere mit künstlicher Intelligenz AI;
wobei das Diagnostic Gate (DG) so ausgebildet ist, dass es unbearbeitete oder unbefundete Studien, insbesondere im DICOM-Datenformat, von einer bilderzeugenden Modalität (M) oder von einem Bildarchivierungs- und Kommunikationssystem (PACS) empfängt, und an die Datenspeicherungs- und Verteileinrichtung (DC), insbesondere an die Servereinheit (S) versendet;
wobei eine unbefundete Studie ein Datensatz ist, mit einer eindeutigen Datensatz-ID, mit oder ohne Patientendaten und mit wenigstens einer Sequenz von Patienten-Bilddaten;
wobei die Servereinheit (S) zur Abspeicherung und Zur-Verfügungsstellung der von dem Diagnostic Gate (DG) empfangenen unbearbeiteten oder unbefundeten Studien, insbesondere im DICOM-Datenformat, und zur Abspeicherung und Zur-Verfügungsstellung der von der Verteileinheit (MDR) empfangenen, bearbeiteten oder befundeten Studien, insbesondere im DICOM-Datenformat, ausgebildet ist;
wobei die Verteileinheit (MDR) so ausgebildet ist, dass sie überprüft, welcher Empfänger (ER, IR, AI) die Berechtigung hat, eine unbefundete oder befundete Studie zu erhalten, diese unbefundete oder befundete Studie an diesen berechtigten Empfänger (ER, IR, AI) sendet und von diesem den Befund oder das Bearbeitungsergebnis zusammen mit der eindeutigen Datensatz-ID zurückerhält;
wobei die Verteileinheit (MDR) so ausgebildet ist,
dass sie aus dem Befund/dem Bearbeitungsergebnis, aus der eindeutigen Datensatz-ID und aus der unbefundeten oder befundeten Studie eine bearbeitete oder befundete Studie erstellt, wobei die bearbeitete oder befundete Studie ein Datensatz ist, mit der eindeutigen Datensatz-ID, mit den Patientendaten, und mit der wenigstens einen Sequenz von Patienten-Bilddaten der unbearbeiteten oder unbefundeten Studie und mit dem vom Empfänger (ER, IR, AI) erstellten Bearbeitungsergebnis oder Befund;
dass sie die bearbeitete oder befundete Studie an die Servereinheit (S) zur Abspeicherung derselben darin sendet, und/oder
dass die bearbeitete oder befundete Studie über das Diagnostic Gate (DG) an die lokale Untersuchungseinrichtung (LU), insbesondere zur Darstellung auf der lokalen Bedienkonsole (K) und/oder zur Abspeicherung in der Bildarchivierungs- und Kommunikationseinheit (PACS) und/oder in dem Radiologieinformationssystem (RIS/KIS) gesendet wird;
oder
wobei die Verteileinheit (MDR) so ausgebildet ist,
dass sie den Befund oder das Bearbeitungsergebnis zusammen mit der eindeutigen Datensatz-ID an die Servereinheit (S) zur Abspeicherung derselben darin sendet, und/oder
dass der Befund oder das Bearbeitungsergebnis zusammen mit der eindeutigen Datensatz-ID über das Diagnostic Gate (DG) an die lokale Untersuchungseinrichtung (LU) gesendet wird,
insbesondere zum Erstellen einer bearbeiteten oder befundeten Studie aus dem Bearbeitungsergebnis oder Befund, aus der eindeutigen Datensatz-ID und aus der unbefundeten oder befundeten Studie, wobei die bearbeitete oder befundete Studie ein Datensatz ist, mit der eindeutigen Datensatz-ID, mit den Patientendaten, und mit der wenigstens einen Sequenz von Patienten-Bilddaten der unbearbeiteten oder unbefundeten Studie und mit der/dem vom Empfänger (ER, IR, AI) erstellten Bearbeitungsergebnis oder Befund, und/oder zur Darstellung auf der lokalen Bedienkonsole K und/oder
insbesondere zur Abspeicherung in dem Bildarchivierungs- und Kommunikationssystem (PACS) und/oder in dem Radiologieinformationssystem (RIS/KIS).

2. Teleradiologisches System nach Anspruch 1,
weiterhin aufweisend wenigstens eine lokale Untersuchungseinrichtung (LU) mit
einer bilderzeugenden Modalität (M);
einem Bildarchivierungs- und Kommunikationssystem (PACS) zur Speicherung von Patienten-Bilddaten, insbesondere im DICOM-Datenformat;
einem Radiologieinformationssystem (RIS/KIS) zur Speicherung von medizinischen und administrativen Daten und zur Prozesssteuerung, insbesondere im HL7-Datenformat; und
einer lokalen Bedienkonsole (K) zur Steuerung der Modalität (M);
wobei das Diagnostic Gate (DG) ebenfalls Bestandteil der lokalen Untersuchungseinrichtung (LU) und so ausgebildet ist, dass es unbearbeitete oder unbefundete Studien, insbesondere im DICOM-Datenformat, von der bilderzeugenden Modalität (M) oder von dem Bildarchivierungs- und Kommunikationssystem (PACS) empfängt, ggf. anonymisiert oder pseudonymisiert, und an die Datenspeicherungs- und Verteileinrichtung (DC), insbesondere an die Servereinheit (S) versendet.

3. Teleradiologisches System nach Anspruch 1 oder 2,
wobei die Verteileinheit (MDR) so ausgebildet ist, dass sie eine Bearbeitung oder einen Befund, nach Erstellung desselben, mit dem Datensatz der unbearbeiteten oder unbefundeten Studie verknüpft, um daraus eine bearbeitete oder befundete Studie zu erstellen.

4. Teleradiologisches System nach einem der vorhergehenden Ansprüche,
wobei zwei oder mehrere lokale Untersuchungseinrichtungen (LU) vorgesehen sind;
wobei die Verteileinheit (MDR) eine Liste von Empfängern (ER, IR, Al), eine Liste von lokalen Untersuchungseinrichtungen (LU) oder deren Diagnostic Gates (DG) und eine Berechtigungsinformation umfasst, welche Empfänger (ER, IR, AI) Studien von welchem Diagnostic Gate (DG) erhalten dürfen; und/oder
wobei die Verteileinheit (MDR) eine Berechtigungsverwaltung durchführt.

5. Teleradiologisches System nach einem der vorhergehenden Ansprüche,
wobei die Datenspeicherungs- und Verteileinrichtung (DC) über wenigstens einen als internen radiologischen Bearbeitungs- oder Befundungsarbeitsplatz (IR) ausgebildeten Empfänger und/oder über einen als automatische Vorbearbeitungs- oder Vorbefundungseinheit (AI) ausgebildeten Empfänger, insbesondere mit künstlicher Intelligenz AI verfügt; und/oder
wobei wenigstens ein als externer radiologischer Bearbeitungs- oder Befundungsarbeitsplatz (ER) ausgebildeter Empfänger entfernt von der Datenspeicherungs- und Verteileinrichtung (DC) vorgesehen ist.

6. Teleradiologisches System nach einem der vorhergehenden Ansprüche,
wobei in dem Fall, dass die Datenspeicherungs- und Verteileinrichtung (DC) über einen als automatische Vorbearbeitungs- oder Vorbefundungseinheit (AI) ausgebildeten Empfänger, insbesondere mit künstlicher Intelligenz AI verfügt, dieser bedienerlos eine Vorbearbeitung oder Vorbefundung durchführt,
wobei wenn die automatische Vorbearbeitungs- oder Vorbefundungseinheit (AI) eine Vorbefundung durchführt, diese wenigstens eine Sequenz von Patienten-Bilddaten einer unbearbeiteten oder unbefundeten Studie automatisiert mit gespeicherten Referenzbildern verglichen wird, und aus dem Vergleichsergebnis einen Vorbefund erstellt,
wobei wenn die automatische Vorbearbeitungs- oder Vorbefundungseinheit (AI) eine Vorbearbeitung durchführt, diese wenigstens eine Sequenz von Patienten-Bilddaten einer unbearbeiteten oder unbefundeten Studie automatisiert vermisst oder automatisiert eine Auswertung oder einen Vergleich mit gespeicherten Referenzbildern durchführt, und ein Vorbearbeitungsergebnis zur Verfügung stellt.

7. Teleradiologisches System nach Anspruch 6,
wobei die Verteileinheit (MDR) so ausgebildet ist,
dass sie aus dem von der automatischen Vorbearbeitungs- oder Vorbefundungseinheit (AI) erstellten Vorbefund/Vorbearbeitungsergebnis, aus der eindeutigen Datensatz-ID und der unbefundeten oder befundeten Studie eine vorbearbeitete oder vorbefundete Studie erstellt, wobei die vorbearbeitete oder vorbefundete Studie ein Datensatz ist, mit der eindeutigen Datensatz-ID, mit oder ohne Patientendaten, mit der wenigstens einen Sequenz von Patienten-Bilddaten der unbearbeiteten oder unbefundeten Studie und mit der/dem von der automatischen Vorbearbeitungs- oder Vorbefundungseinheit (AI) erstellten Vorbefund/Vorbearbeitungsergebnis;
dass sie die vorbearbeitete oder vorbefundete Studie an die Servereinheit (S) zur Abspeicherung derselben darin sendet, und/oder
dass die vorbearbeitete oder vorbefundete Studie über das Diagnostic Gate (DG) an die lokale Untersuchungseinrichtung (LU), insbesondere an die lokale Bedienkonsole (K) zur Endbearbeitung und/oder Endbefundung und/oder zur Abspeicherung in dem Bildarchivierungs- und Kommunikationssystem (PACS) und/oder in dem Radiologieinformationssystem (RIS/KIS) gesendet wird.

8. Teleradiologisches System nach Anspruch 6 oder 7,
wobei die Verteileinheit (MDR) so ausgebildet ist,
dass sie den/das von der automatischen Vorbearbeitungs- oder Vorbefundungseinheit (AI) erstellte(n) Vorbefund/Vorbearbeitungsergebnis zusammen mit der eindeutigen Datensatz-ID an die Servereinheit (S) zur Abspeicherung derselben darin sendet, und/oder
dass der Vorbefund/ das Vorbearbeitungsergebnis zusammen mit der eindeutigen Datensatz-ID über das Diagnostic Gate (DG) an die lokale Untersuchungseinrichtung (LU) gesendet wird,
wobei in der lokalen Untersuchungseinrichtung (LU) eine vorbearbeitete oder vorbefundete Studie aus dem Vorbefund/Vorbearbeitungsergebnis, aus der eindeutigen Datensatz-ID und der unbefundeten oder befundeten Studie erstellt und/oder in dem Bildarchivierungs- und Kommunikationssystem (PACS) und/oder in dem Radiologieinformationssystem (RIS/KIS) abgespeichert wird,
wobei die vorbearbeitete oder vorbefundete Studie ein Datensatz ist, mit der eindeutigen Datensatz-ID, mit oder ohne Patientendaten, mit der wenigstens einen Sequenz von Patienten-Bilddaten der unbearbeiteten oder unbefundeten Studie und mit der/dem von der automatischen Vorbearbeitungs- oder Vorbefundungseinheit (AI) erstellten Vorbefund/Vorbearbeitungsergebnis; und/oder
wobei wenigstens der Vorbefund/das Vorbearbeitungsergebnis auf der lokalen Bedienkonsole (K) angezeigt wird und eine Endbefundung durchführbar ist.

9. Teleradiologisches System nach einem der Ansprüche 6 bis 8,
wobei die lokale Untersuchungseinrichtung (LU), insbesondere die lokale Bedienkonsole (K) so ausgebildet ist,
dass sie eine Endbearbeitung oder einen Endbefund, nach Erstellung desselben, mit dem Datensatz der vorbearbeiteten oder vorbefundeten Studie verknüpft, um daraus eine endbearbeitete oder endfundete Studie zu erstellen, und
dass sie die endbearbeitete oder endbefundete Studie über das Diagnostic Gate (DG) an die Servereinheit (S) der Datenspeicherungs- und Verteileinrichtung (DC) zur Abspeicherung derselben darin versendet, und/oder
dass sie die endbearbeitete oder endbefundete Studie in dem Bildarchivierungs- und Kommunikationssystem (PACS) und/oder in dem Radiologieinformationssystem (RIS/KIS) abspeichert.

10. Teleradiologisches System nach einem der vorhergehenden Ansprüche,
wobei die Servereinheit (S), insbesondere Serverfarm, zur Abspeicherung und Zur-Verfügungsstellung der unbearbeiteten oder unbefundeten Studien, und/oder der bearbeiteten oder befundeten Studien, und/oder der vorbearbeiteten oder vorbefundeten Studien und/oder der endbearbeiteten oder endbefundeten Studien, insbesondere im DICOM- und/oder HL7-Datenformat für Befunde/Zuweisungen, ausgebildet ist,
wobei die Servereinheit (S), insbesondere Serverfarm, insbesondere so ausgebildet ist, dass sie Zugriff auf unbearbeitete oder unbefundete Studien, und/oder auf bearbeitete oder befundete Studien, und/oder auf vorbearbeitete oder vorbefundete Studien und/oder auf endbearbeitete oder endbefundete Studien, insbesondere im DICOM- und/oder HL7-Datenformat für Befunde/Zuweisungen, von einem Arbeitsplatz eines zuweisenden Arztes (AA) oder anderen Befunders oder Behandlers oder Bearbeiters oder einem Patientenarbeitsplatz (AP) aus erlaubt.

11. Teleradiologisches System nach einem der vorhergehenden Ansprüche,
wobei das Diagnostic Gate (DG) oder die Verteileinheit (MDR) so ausgebildet ist, dass es unbearbeitete oder unbefundete Studien anonymisiert oder pseudonymisiert;
wobei das Diagnostic Gate (DG) oder die Verteileinheit (MDR) so ausgebildet ist, dass beim Pseudonymisieren einer unbearbeiteten oder unbefundeten Studie die Patientendaten und die wenigstens eine Sequenz von Patienten-Bilddaten verschlüsselt werden, unter unverschlüsselter Beibehaltung der eindeutigen Datensatz-ID.

12. Teleradiologisches System nach einem der vorhergehenden Ansprüche,
wobei die unbearbeitete oder unbefundete oder die endbearbeitete oder endbefundete Studie vor dem Versenden durch das Diagnostic Gate (DG) verschlüsselt, und anschließend durch die Servereinheit (S) entschlüsselt wird; und/oder
wobei die bearbeitete oder befundete Studie oder die vorbearbeitete oder vorbefundete Studie vor dem Versenden an das Diagnostic Gate (DG) durch die Servereinheit (S) verschlüsselt, und anschließend durch das Diagnostic Gate (DG) entschlüsselt wird; und/oder
wobei die unbearbeitete oder unbefundete Studie vor dem Versenden der unbearbeiteten oder unbefundeten Studie an den wenigstens einen als externen radiologischen Befundungsarbeitsplatz (ER) ausgebildeten Empfänger verschlüsselt, und durch diesen entschlüsselt wird;
wobei die bearbeitete oder befundete Studie vor dem Zurücksenden durch den wenigstens einen als externen radiologischen Befundungsarbeitsplatz (ER) ausgebildeten Empfänger verschlüsselt, und anschließend entschlüsselt wird.

13. Teleradiologisches System nach einem der vorhergehenden Ansprüche,
wobei die bilderzeugende Modalität (M) und/oder das Bildarchivierungs- und Kommunikationssystem (PACS) zur Speicherung von Patienten-Bilddaten so ausgebildet oder programmiert ist/sind, dass die unbearbeiteten oder unbefundeten Studien automatisch an das Diagnostic Gate (DG) versendet werden;
wobei die unbearbeiteten oder unbefundeten Studien von einem Benutzer, insbesondere der lokalen Bedienkonsole (K), manuell an das Diagnostic Gate (DG) versendet werden.

14. Verfahren zur teleradiologischen Fernbefundung und Fernbearbeitung sowie zum teleradiologischen Datenmanagement,
wobei
ein Diagnostic Gate (DG) mit einer Schnittstelle zu einer bilderzeugenden Modalität (M) einer lokalen Untersuchungseinrichtung (LU) oder zu einem Bildarchivierungs- und Kommunikationssystem (PACS) zur Speicherung von Patienten-Bilddaten, insbesondere im DICOM-Datenformat, einer lokalen Untersuchungseinrichtung (LU) oder zu einem Radiologieinformationssystem (RIS/KIS) zur Speicherung von medizinischen und administrativen Daten und zur Prozesssteuerung, insbesondere im HL7-Datenformat;
eine Datenspeicherungs- und Verteileinrichtung (DC) mit
einer Servereinheit (S), insbesondere einer Serverfarm; und
einer Verteileinheit (MDR), insbesondere einem Medical Data Router; und
wenigstens ein Empfänger (ER, IR, AI), insbesondere ein radiologischer Befundungsarbeitsplatz (ER, IR) oder eine automatische Vorbefundungs- oder Bearbeitungseinheit (AI), insbesondere mit künstlicher Intelligenz AI;
vorgesehen sind;
wobei das Verfahren die folgenden Schritte aufweist:
Empfangen einer unbearbeiteten oder unbefundeten Studie, insbesondere im DICOM-Datenformat, von einer bilderzeugenden Modalität (M) oder von einem Bildarchivierungs- und Kommunikationssystem (PACS) durch ein Diagnostic Gate (DG), wobei eine unbearbeitete oder unbefundete Studie ein Datensatz ist, mit einer eindeutigen Datensatz-ID, mit oder ohne Patientendaten und mit wenigstens einer Sequenz von Patienten-Bilddaten;
gegebenenfalls Anonymisieren oder Pseudonymisieren der unbearbeiteten oder unbefundeten Studie;
Versenden der unbearbeiteten oder unbefundeten Studie an die Datenspeicherungs- und Verteileinrichtung (DC), insbesondere an eine Servereinheit (S);
Abspeichern der unbearbeiteten oder unbefundeten Studie in der Servereinheit (S);
Überprüfen durch die Verteileinheit (MDR), welcher Empfänger (ER, IR, AI) die Berechtigung hat, diese unbefundete Studie zu erhalten, und Versenden dieser unbearbeiteten oder unbefundeten Studie an diesen berechtigten Empfänger (ER, IR, AI);
Empfangen des Befunds, durch die Verteileinheit (MDR), zusammen mit der eindeutigen Datensatz-ID von dem Empfänger (ER, IR, AI);
Erstellen einer bearbeiteten oder befundeten Studie, durch die Verteileinheit (MDR), aus dem Befund, der eindeutigen Datensatz-ID und der unbefundeten oder befundeten Studie, wobei die bearbeitete oder befundete Studie ein Datensatz ist, mit der eindeutigen Datensatz-ID, mit den Patientendaten, und mit der wenigstens einen Sequenz von Patienten-Bilddaten der unbearbeiteten oder unbefundeten Studie und mit der/dem vom Empfänger (ER, IR, AI) erstellten Bearbeitungsergebnis oder Befund;
Versenden der bearbeiteten oder befundeten Studie an die Servereinheit (S) zur Abspeicherung derselben darin sendet, und/oder
Versenden der bearbeiteten oder befundeten Studie über das Diagnostic Gate (DG) an die lokale Untersuchungseinrichtung (LU), insbesondere zur Darstellung auf der lokalen Bedienkonsole (K) und/oder zur Abspeicherung in der Bildarchivierungs- und Kommunikationseinheit (PACS) und/oder in dem Radiologieinformationssystem (RIS/KIS); oder
Versenden, durch die Verteileinheit (MDR), des Befunds oder des Bearbeitungsergebnisses zusammen mit der eindeutigen Datensatz-ID an die Servereinheit (S) zur Abspeicherung derselben darin, und/oder
Versenden des Befunds oder des Bearbeitungsergebnisses zusammen mit der eindeutigen Datensatz-ID über das Diagnostic Gate (DG) an die lokale Untersuchungseinrichtung (LU), insbesondere zum Erstellen einer bearbeiteten oder befundeten Studie aus dem Befund oder dem Bearbeitungsergebnis, aus der eindeutigen Datensatz-ID und aus der unbefundeten oder befundeten Studie, wobei die bearbeitete oder befundete Studie ein Datensatz ist, mit der eindeutigen Datensatz-ID, mit den Patientendaten, und mit der wenigstens einen Sequenz von Patienten-Bilddaten der unbearbeiteten oder unbefundeten Studie und mit der/dem vom Empfänger (ER, IR, AI) erstellten Bearbeitungsergebnis oder Befund, und/oder zur Darstellung auf der lokalen Bedienkonsole (K) und/oder zur Abspeicherung in dem Bildarchivierungs- und Kommunikationssystem (PACS) und/oder in dem Radiologieinformationssystem (RIS/KIS).

15. Verfahren nach Anspruch 14,
wobei in dem Fall, dass die Datenspeicherungs- und Verteileinrichtung (DC) über eine als automatische Vorbearbeitungs- oder Vorbefundungseinheit (AI) ausgebildeten Empfänger, insbesondere mit künstlicher Intelligenz AI verfügt, dieser bedienerlos eine Vorbearbeitung oder Vorbefundung durchführt,
wobei die Verteileinheit (MDR) aus dem von der automatischen Vorbearbeitungs- oder Vorbefundungseinheit (AI) erstellten Vorbefund/Vorbearbeitungsergebnis, aus der eindeutigen Datensatz-ID und der unbefundeten oder befundeten Studie eine vorbearbeitete oder vorbefundete Studie erstellt, wobei die vorbearbeitete oder vorbefundete Studie ein Datensatz ist, mit der eindeutigen Datensatz-ID, mit oder ohne Patientendaten, mit der wenigstens einen Sequenz von Patienten-Bilddaten der unbearbeiteten oder unbefundeten Studie und mit der/dem von der automatischen Vorbearbeitungs- oder Vorbefundungseinheit (AI) erstellten Vorbefund/Vorbearbeitungsergebnis;
wobei die Verteileinheit (MDR) vorbearbeitete oder vorbefundete Studie an die Servereinheit (S) zur Abspeicherung derselben darin sendet, und/oder
wobei die vorbearbeitete oder vorbefundete Studie über das Diagnostic Gate (DG) an die lokale Untersuchungseinrichtung (LU), insbesondere an die lokale Bedienkonsole (K) zur Endbearbeitung und/oder Endbefundung und/oder zur Abspeicherung in dem Bildarchivierungs- und Kommunikationssystem (PACS) und/oder in dem Radiologieinformationssystem (RIS/KIS) gesendet wird;
oder
wobei die Verteileinheit (MDR) den/das von der automatischen Vorbearbeitungs- oder Vorbefundungseinheit (AI) erstellte(n) Vorbefund/Vorbearbeitungsergebnis zusammen mit der eindeutigen Datensatz-ID an die Servereinheit (S) zur Abspeicherung derselben darin sendet, und/oder
wobei der Vorbefund/ das Vorbearbeitungsergebnis zusammen mit der eindeutigen Datensatz-ID über das Diagnostic Gate (DG) an die lokale Untersuchungseinrichtung (LU) gesendet wird, und/oder
wobei in der lokalen Untersuchungseinrichtung (LU) eine vorbearbeitete oder vorbefundete Studie aus dem Vorbefund/Vorbearbeitungsergebnis, aus der eindeutigen Datensatz-ID und der unbefundeten oder befundeten Studie erstellt und/oder in dem Bildarchivierungs- und Kommunikationssystem (PACS) und/oder in dem Radiologieinformationssystem (RIS/KIS) abgespeichert wird, und/oder
wobei die vorbearbeitete oder vorbefundete Studie ein Datensatz ist, mit der eindeutigen Datensatz-ID, mit oder ohne Patientendaten, mit der wenigstens einen Sequenz von Patienten-Bilddaten der unbearbeiteten oder unbefundeten Studie und mit der/dem von der automatischen Vorbearbeitungs- oder Vorbefundungseinheit (AI) erstellten Vorbefund/Vorbearbeitungsergebnis; und/oder
wobei wenigstens der Vorbefund/das Vorbearbeitungsergebnis auf der lokalen Bedienkonsole (K) angezeigt wird und eine Endbefundung durchführbar ist;
und/oder
wobei die lokale Untersuchungseinrichtung (LU), insbesondere die lokale Bedienkonsole (K), eine Endbearbeitung oder einen Endbefund, nach Erstellung desselben, mit dem Datensatz der vorbearbeiteten oder vorbefundeten Studie verknüpft, um daraus eine endbearbeitete oder endfundete Studie zu erstellen, und
wobei die lokale Untersuchungseinrichtung (LU) die endbearbeitete oder endbefundete Studie über das Diagnostic Gate (DG) an die Servereinheit (S) der Datenspeicherungs- und Verteileinrichtung (DC) zur Abspeicherung derselben darin versendet, und/oder
wobei die lokale Untersuchungseinrichtung (LU) die endbearbeitete oder endbefundete Studie in dem Bildarchivierungs- und Kommunikationssystem (PACS) und/oder in dem Radiologieinformationssystem (RIS/KIS) abspeichert.
